# EUROPEAN PATENT APPLICATION

(11) **EP 2 644 101 A1**
(43) Date of publication of application: **02.10.2013**
(21) Application number: 13161648.4
(22) Date of filing: 28.03.2013
(51) Int. Cl.: A61B 8/00, B06B 1/06

(54) **Ultrasonic probe, electronic instrument, and ultrasonic diagnostic device**

(30) Priority: 30.03.2012 JP 2012078674
(71) Applicant: Seiko Epson Corporation, Tokyo 163-0811 (JP)
(72) Inventor: Nakamura, Tomoaki, Suwa-shi Nagano, 392-8502 (JP); Nishiwaki, Tsutomu, Suwa-shi Nagano, 392-8502 (JP); Onishi, Yasunori, Suwa-shi Nagano, 392-8502 (JP); Miyazawa, Takao, Suwa-shi Nagano, 392-8502 (JP)
(74) Representative: HOFFMANN EITLE

(57) **Abstract**

An ultrasonic probe (12) includes an element chip (22), a flexible wiring member (23) and a control circuit (24). The element chip includes a substrate (31) forming a plurality of openings (48) arranged in an array pattern and a plurality of ultrasonic transducer elements (33) disposed at the openings. The flexible wiring member is connected to the element chip, and forming at least a part of an annular body (21) surrounding a space. The control circuit is connected to the flexible wiring member and electrically connected to the ultrasonic transducer elements via the flexible wiring member.

## Description

### BACKGROUND

### Technical Field

The present invention relates to an ultrasonic probe, and an electronic instrument, an ultrasonic diagnostic device, and the like that use the ultrasonic probe.

### Related Art

An ultrasonic probe, that is, a finger probe for being mounted on a human's finger has been widely known (for example, see Japanese Laid-open Patent Publication No. 2002-291746). In such a finger probe, a single ultrasonic transducer element or an element array formed with an arrangement of a large number of bulk-type ultrasonic transducer elements is used. Scanning of ultrasonic beams can be achieved by using such an element array. A cross-sectional image can be formed corresponding to the scanning. A large number of signal lines are extracted from the element array for achieving scanning.

### SUMMARY

When an element array is used, a large number of signal lines are bundled and extracted by a cable. For example, when the element array is disposed on a ball of a finger, the cable crosses the first joint or the second joint of the finger on a palm side along the finger. Since the signal lines are bundled in the cable, the rigidity of the cable is inevitably increased. Therefore, the bend of the finger is restricted due to use of the finger probe. Further, when the ultrasonic probe is mounted on a palm and the like, the ultrasonic probe can be brought into contact with an object like a hand, which achieves great user-friendliness. Preferably, even in such a case, restricting movement such as the bend of a hand due to the rigidity of the cable should be avoided.

According to at least one aspect of the present invention, an ultrasonic probe that sufficiently allows the bend of a finger or a hand can be provided.

According to one aspect of the present invention, an ultrasonic probe includes an element chip, a flexible wiring member and a control circuit. The element chip includes a substrate forming a plurality of openings arranged in an array pattern and a plurality of ultrasonic transducer elements disposed at the openings. The flexible wiring member is connected to the element chip, and forming at least a part of an annular body surrounding a space. The control circuit is connected to the flexible wiring member and electrically connected to the ultrasonic transducer elements via the flexible wiring member.

The flexible wiring member is not extracted along a two-dimensional plane that is continuous with the element chip, but is extracted three-dimensionally along a space so as to surround the space. As a result, it is possible to reduce the size with respect to the two-dimensional plane. The ultrasonic probe can be supported by a supporting body that enters the space inside the annular body. The bend of the supporting body can be allowed because there is no bundle of signal lines along the two-dimensional plane that is continuous with the element chip.

According to another aspect of the present invention, the ultrasonic probe further preferably includes a control circuit chip including the control circuit. The flexible wiring member preferably includes a first flexible wiring section connected to the element chip and extending from the element chip in a first direction, and a second flexible wiring section connected to the element chip and extending from the element chip in a second direction that is opposite to the first direction. The element chip, the first flexible wiring section, the second flexible wiring section, and the control circuit chip preferably collectively form the annular body.

The first flexible wiring section and the second flexible wiring section are not extracted along a two-dimensional plane that is continuous with the element chip, but are extracted three-dimensionally along a space so as to surround the space. As a result, it is possible to reduce the size with respect to the two-dimensional plane. The ultrasonic probe can be supported by a supporting body that enters the space inside the annular body. The bend of the supporting body can be allowed because there is no bundle of signal lines extracted along the two-dimensional plane that is continuous with the element chip.

The ultrasonic probe may further include a tubular body member made of an elastic film body and defining a tubular internal space passing through the space surrounded by the annular body to form an entrance space for a finger, with the annular body constituting a finger band. The ultrasonic probe may be constructed in a shape of a fingerstall. A finger can enter the internal space of the tubular body. As a result, the annular body can be mounted on a fingertip (for example, a distal joint, or a distal joint and a proximal joint). The tubular body can be closely attached to a finger due to the action of an elastic force. Consequently, the element chip can be fixed to a fingertip. The bend of a finger can be allowed because there is no bundle of signal lines along the two-dimensional plane that is continuous with the element chip.

The annular body may constitute a finger band, and at least a part of the annular body may convey a contact state to a finger. The annular body can be mounted on a fingertip (for example, a distal joint, or a distal joint and a proximal joint). A feel of an object can be transmitted to a fingertip through the ultrasonic probe. A user can sense a feel of a diseased part while mounting the ultrasonic probe.

The ultrasonic probe may further include a reinforcing member fixed to a second surface of the substrate, which is an opposite side of a first surface where the ultrasonic transducer elements are disposed, for reinforcing the substrate. The ultrasonic transducer elements can be made thin. The ultrasonic transducer elements can be made on a thin substrate. Even if the reinforcing member is fixed to the substrate, the ultrasonic transducer element chip can be made thin. In addition, since the reinforcing member is fixed to the second surface of the substrate, the strength of the substrate can be reinforced in a thickness direction of the substrate.

Internal spaces of the openings may be continuous with an external space of the substrate. The internal spaces of the openings are connected to the external space of the substrate, and ventilation can be secured between the internal spaces of the openings and the outside of the internal spaces. Consequently, the internal spaces of the openings are not sealed. The internal spaces of the openings can easily follow change in the ambient pressure. It is thus possible to securely prevent the ultrasonic transducer elements from being damaged. If the internal spaces of the openings are hermetically sealed, there is a concern that the ultrasonic transducer element will be damaged due to change in the pressure.

The reinforcing member may be bonded to a partition wall section of the substrate between the openings in at least one bonding region. When the partition wall section of the substrate is bonded to the reinforcing member, the movement of the partition wall section is restricted by the reinforcing member. Thus, vibration of the partition wall section can be prevented. As a result, crosstalk between the ultrasonic transducer elements can be prevented. Further, when the movement of the partition wall section is restricted, vibration of the partition wall section can be prevented from acting on ultrasonic vibration of the ultrasonic transducer elements. Then, ultrasonic vibration in a clear vibration mode can be obtained in the ultrasonic transducer elements. Consequently, when vibration of the partition wall section is avoided, the amplitude of ultrasonic vibration can be prevented from being decreased.

The control circuit may include a multiplexer configured to control traffic of signals between a first prescribed number of first signal lines extracted from the ultrasonic transducer elements and a second prescribed number of second signal lines, the second prescribed number being smaller than the first prescribed number. Therefore, the second signal lines are extracted from the ultrasonic probe via the multiplexer and the first signal lines. Compared to the case where the first signal lines are extracted directly from the ultrasonic probe, the number of the extracted signal lines is decreased. As a result, the size of a connector or a cable connected to the ultrasonic probe can be reduced. Consequently, size reduction of the ultrasonic probe can be promoted, and the bend of the supporting body is not hindered irrespective of wiring of a cable.

The ultrasonic probe may be incorporated in an electronic instrument. The electronic instrument may be provided with the ultrasonic probe, and a processing circuit connected to the ultrasonic probe and configured to process output signals of the ultrasonic transducer elements.

The ultrasonic probe may be incorporated in an ultrasonic diagnostic device. The ultrasonic diagnostic device may be provided with the ultrasonic probe, a processing circuit that is connected to the ultrasonic probe and configured to process output signals of the ultrasonic transducer elements to generate an image, and a display device that displays the image.

The ultrasonic probe may be incorporated in an ultrasonic probe set. The ultrasonic probe set may be provided with the ultrasonic probe, and a relay device connected to the ultrasonic probe and has a communication circuit connected to the control circuit.

The relay device may include at least a part of the control circuit. When the relay device takes a part of the function of the control circuit, the size of the ultrasonic probe can further be reduced.

The ultrasonic probe set may be incorporated in an electronic instrument. The electronic instrument may be provided with the ultrasonic probe set, and a processing circuit wirelessly connected to the communication circuit and configured to process output signals of the ultrasonic transducer elements.

The ultrasonic probe set may be incorporated in an ultrasonic diagnostic device. The ultrasonic diagnostic device may be provided with the ultrasonic probe set, a processing circuit wirelessly connected to the communication circuit and configured to process output signals of the ultrasonic transducer elements to generate an image, and a display device configured to display the image.

### BRIEF DESCRIPTION OF THE DRAWINGS

Referring now to the attached drawings which form a part of this original disclosure:

Fig. 1 is a view schematically showing an example of an ultrasonic detection device, that is, an ultrasonic diagnostic device according to one embodiment of the present invention.

Fig. 2 is an enlarged vertical sectional view of an ultrasonic probe.

Fig. 3 is an enlarged perspective view schematically showing a configuration of an annular body unit according to a first embodiment of the present invention.

Fig. 4 is an enlarged plan view of an element chip.

Fig. 5 is a sectional view along line 5-5 of Fig. 4.

Fig. 6 is a plan view of a reinforcing plate showing grooves.

Fig. 7 is a partially enlarged plan view of Fig. 6.

Fig. 8 is a block diagram schematically showing a circuit configuration of the ultrasonic diagnostic device.

Fig. 9 is an enlarged perspective view schematically showing a configuration of an annular body unit according to a second embodiment of the present invention.

Fig. 10 is an enlarged perspective view schematically showing a configuration of an annular body unit according to a third embodiment of the present invention.

Fig. 11 is an enlarged perspective view schematically showing a configuration of an annular body unit according to a fourth embodiment of the present invention.

Fig. 12 is a block diagram schematically showing a circuit configuration according to another embodiment.

Fig. 13 is a view schematically showing an ultrasonic probe according to another embodiment.

Fig. 14 is a view schematically showing an ultrasonic probe according to another embodiment.

Fig. 15 is a view schematically showing an ultrasonic probe according to another embodiment.

### DETAILED DESCRIPTION OF EXEMPLARY EMBODIMENTS

Next, embodiments of the present invention will be explained with reference to the attached drawings. The embodiments explained below shall not be construed as unreasonably limiting the subject matter of the present invention described in the claims, and all the elements explained in the embodiments are not necessarily essential to the solving means of the present invention.

### (1) Overall Configuration of Ultrasonic Detection Device

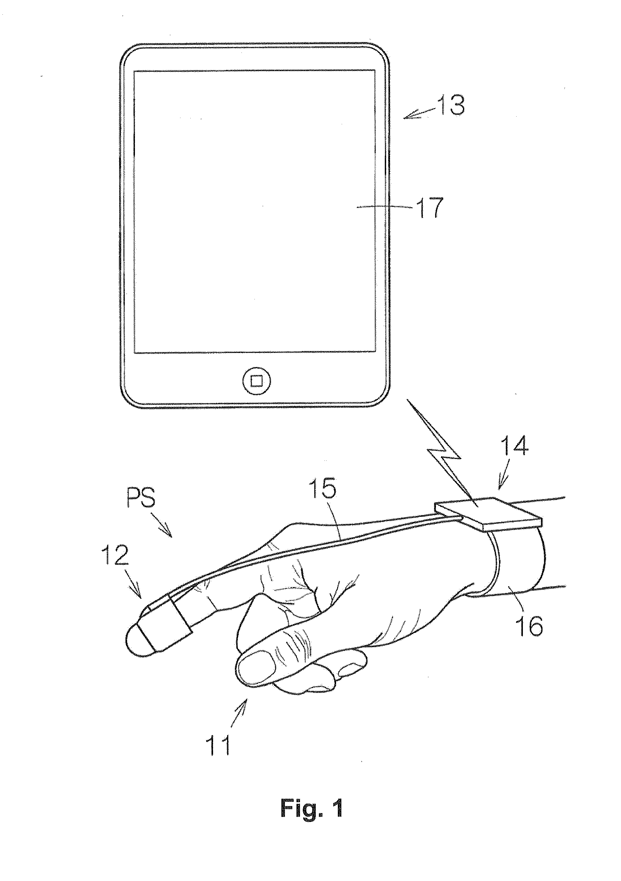

Fig. 1 schematically shows a configuration of an ultrasonic diagnostic device 11 as an example of an electronic instrument according to an embodiment of the present invention. The ultrasonic diagnostic device 11 is provided with an ultrasonic probe 12, a device terminal 13, and a relay device 14. The ultrasonic probe 12 and the relay device 14 form a single ultrasonic probe set PS. The ultrasonic probe set PS can be mounted on an operator's hand.

The ultrasonic probe 12 is constructed in a shape of a fingerstall or finger cap. The ultrasonic probe 12 and the relay device 14 are connected to each other, for example, through a cable 15. The ultrasonic probe 12 and the relay device 14 communicate an electric signal through the cable 15. The relay device 14 is provided with a wrist band 16. The relay device 14 can be mounted on an operator's wrist by the wrist band 16. The device terminal 13 is connected to the relay device 14 wirelessly. The relay device 14 and the device terminal 13 communicate an electric signal through wireless communication. A display panel 17 is incorporated in the device terminal 13. A screen of the display panel 17 is exposed on a surface of the device terminal 13. As described later, in the device terminal 13, an image is generated based on ultrasonic waves detected with the ultrasonic probe 12. Imaged detection results are displayed on the screen of the display panel 17. A user interface can be constructed in the device terminal 13 based on a touch panel or an input pad.

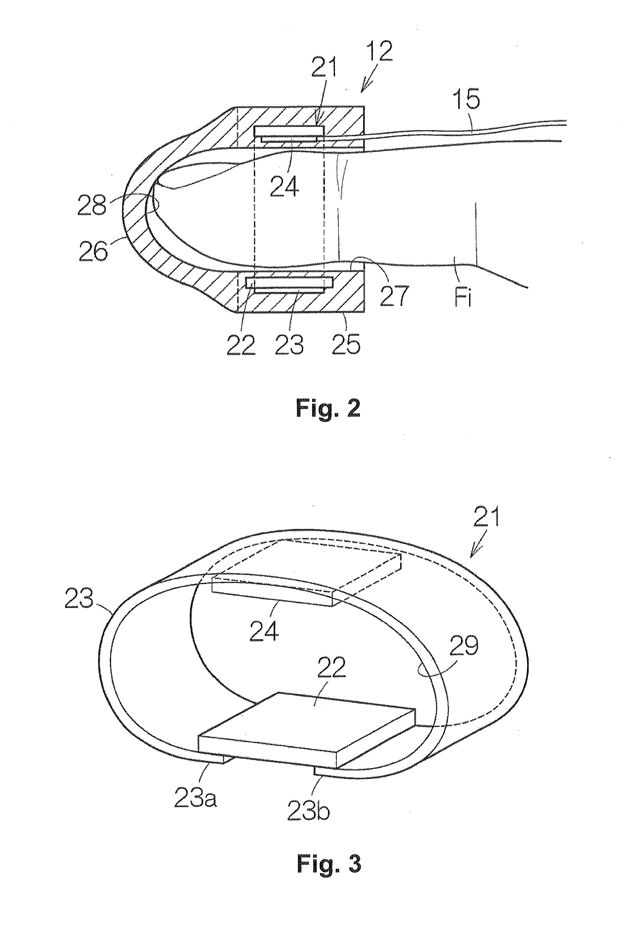

As shown in Fig. 2, the ultrasonic probe 12 has an annular body unit 21. The annular body unit 21 has an element chip 22, a flexible printed substrate 23, and a control circuit 24. The flexible printed substrate 23 is connected to the element chip 22. The control circuit 24 is connected to the flexible printed substrate 23. Here, the control circuit 24 can be formed as an IC chip (one example of a control circuit chip). The IC chip can be mounted, for example, on a surface of the flexible printed substrate 23. The cable 15 is connected to the flexible printed substrate 23. The control circuit 24 is electrically connected to the element chip 22 through signal lines on the flexible printed substrate 23. Similarly, the control circuit 24 is electrically connected to the cable 15 through the signal lines on the flexible printed substrate 23. Here, the flexible printed substrate 23 is used as a flexible wiring member. Alternatively, as the flexible wiring member, an annular body formed with a supporting member that supports a conductive line in the same manner as the flexible printed substrate, or an annular body formed with an electric wire in which a conductive line is covered with an insulating body can be used.

The ultrasonic probe 12 has a tubular body 25 (one example of a tubular body member) and a tip end body 26. The tubular body 25 is made of an elastic film body. As the material for the film body, for example, a resin material having flexibility can be used. The tubular body 25 forms an internal space 27 of a columnar or tubular shape. The internal space 27 of a columnar shape serves as an entrance space for a finger Fi. The annular body unit 21 is embedded into the film of the tubular body 25. Consequently, the annular body unit 21 can be constructed as a finger band.

The tip end body 26 is connected to an end of the tubular body 25. The tip end body 26 is made of an elastic film body similarly to the tubular body 25. The tip end body 26 is formed into a dome shape. An internal space 28 of a hemispherical shape is continuous with the internal space 27 of the tubular body 25. Consequently, an end of the internal space 27 is blocked with the tip end body 26. The internal space 28 of a hemispherical shape can serve as an entrance space for a tip end of the finger Fi. The tubular body 25 and the tip end body 26 can be formed integrally as a single element.

Fig. 3 schematically shows the annular body unit 21 according to a first embodiment of the present invention. In the annular body unit 21, the element chip 22 and the flexible printed substrate 23 collectively form an annular body. The element chip 22 and the flexible printed substrate 23 surround a space 29. A first end 23a of the flexible printed substrate 23 is connected to one end of the element chip 22, and a second end 23b of the flexible printed substrate 23 is connected to the other end of the element chip 22. The internal space 27 of the tubular body 25 passes through the space 29 of the annular body. Accordingly, the finger Fi of an operator enters the space 29 of the annular body.

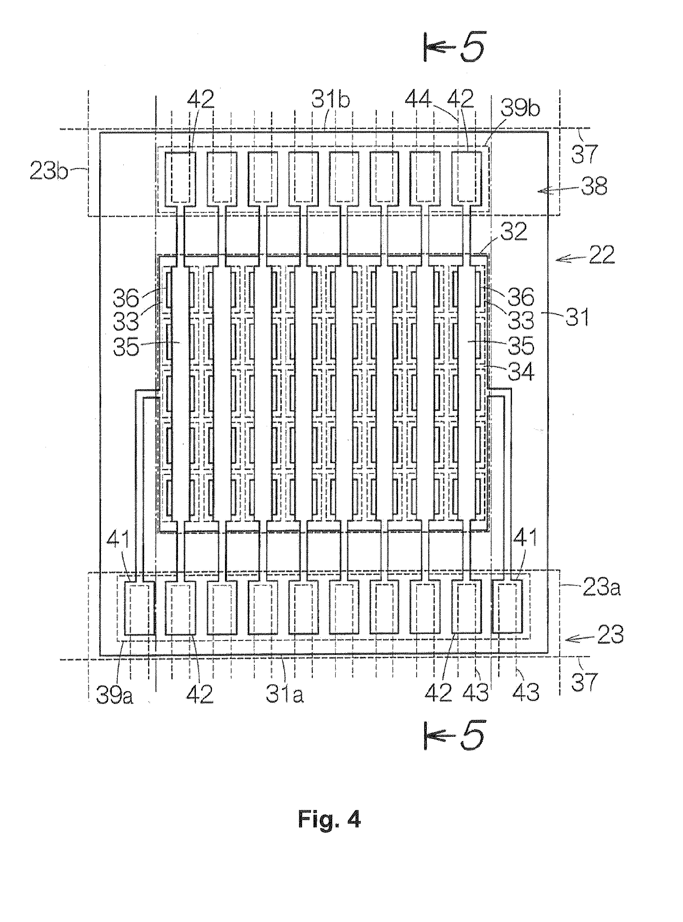

Fig. 4 schematically shows a plan view of the element chip 22. The element chip 22 is provided with a substrate 31. An element array 32 is formed on the substrate 31. The element array 32 is constructed with an arrangement of a plurality of ultrasonic transducer elements (hereinafter referred to as "elements") 33. The arrangement is formed in a matrix having a plurality of columns and a plurality of rows. Each element 33 is constructed of a lower electrode 34, an upper electrode 35, and a piezoelectric film 36. The lower electrode 34 is arranged in common with respect to the elements 33 of the entire matrix. The upper electrode 35 is arranged in common with respect to the elements 33 of each column. The piezoelectric film 36 is sandwiched between the lower electrode 34 and the upper electrode 35 in each element 33. Power distribution to the elements 33 is switched per column. Line scanning or sector scanning is achieved corresponding to such switching of power distribution. Since the elements 33 in one column output ultrasonic waves at the same time, the number of the elements 33 in one column, that is, the row number of the arrangement can be determined based on the output level of ultrasonic waves. For example, the row number may be set to be around 10-15. In the drawing, five rows are illustrated for simplicity. The column number of the arrangement can be determined based on the extent of an area to be scanned. For example, the column number may be set to be 128 or 256. In the drawing, eight columns are illustrated for simplicity. Regarding the arrangement, a zigzag pattern may be used. In the zigzag pattern, a group of the elements 33 in an even column may be displaced with respect to a group of the elements 33 in an odd column by one-half of the row pitch. The number of the elements 33 in one of an odd column and an even column may be smaller than the number of the elements 33 in the other of an odd column and an even column by one. Further, the role of the lower electrode 34 and the role of the upper electrode 35 may be switched. Specifically, the upper electrode may be connected in common to the elements 33 of the entire matrix, and the lower electrode may be connected in common to the elements 33 in each column of the arrangement.

The outer edge of the substrate 31 has a first side 31a and a second side 31b that are opposed and partitioned by a pair of straight lines 37 parallel to each other. A peripheral region 38 extends between the outline of the element array 32 and the outer edge of the substrate 31. In the peripheral region 38, a first terminal array 39a of one line is formed along the first side 31a in parallel to the first side 31a, and a second terminal array 39b of one line is formed along the second side 31b in parallel to the second side 31b. The first terminal array 39a is constructed of a pair of lower electrode terminals 41 and a plurality of upper electrode terminals 42. The second terminal array 39b is constructed of a plurality of upper electrode terminals 42. The lower electrode terminals 41 are connected to the lower electrodes 34 of the elements 33 in common with respect to the entire matrix. The upper electrode terminals 42 are connected to the upper electrodes 35 of the elements 33 in common with respect to each column. Here, the upper electrode terminals 42 of the first terminal array 39a and the upper electrode terminals 42 of the second terminal array 39b are alternately connected to the columns of the upper electrodes 35. When the terminals are extracted in opposite directions alternately with respect to each column, a space of two columns can be used for forming each terminal, and a sufficient size of each terminal can be achieved compared to the case where the terminals are extracted in the same direction. However, the upper electrode terminals 42 of the first terminal array 39a and the upper electrode terminals 42 of the second terminal array 39b may be connected concurrently to each column. When current is supplied to the upper electrodes 35 from both directions, the influence of a voltage drop can be reduced. Here, the outer edge of the substrate 31 is formed into a rectangular shape. The outer edge of the substrate 31 may be a square or a trapezoid.

The first end 23a of the flexible printed substrate 23 covers the first terminal array 39a. Signal lines 43 are formed at the first end 23a of the flexible printed substrate 23 corresponding to the lower electrode terminals 41 and the upper electrode terminals 42, respectively. The signal lines 43 are respectively opposed to the lower electrode terminals 41 and the upper electrode terminals 42, and respectively bonded thereto. The lower electrode terminals 41 and the upper electrode terminals 42 of the first terminal array 39a are respectively connected to the control circuit 24 by the signal lines 43. Similarly, the second end 23b of the flexible printed substrate 23 covers the second terminal array 39b. Signal lines 44 are formed at the second end 23b of the flexible printed substrate 23 corresponding to the upper electrode terminals 42, respectively. The signal lines 44 are respectively opposed to the upper electrode terminals 42, and respectively bonded thereto. The upper electrode terminals 42 of the second terminal array 39b are respectively connected to the control circuit 24 by the signal lines 44.

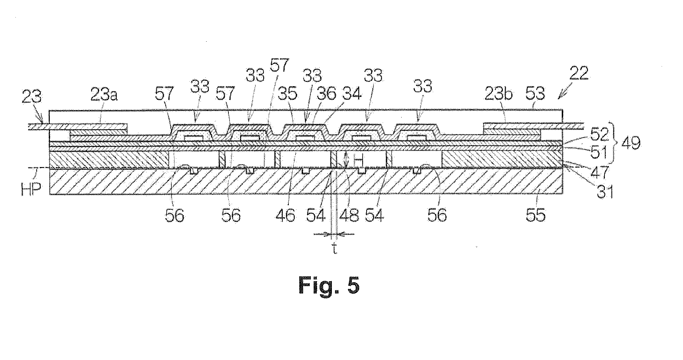

As shown in Fig. 5, each of the elements 33 has a vibrating film 46. In order to construct the vibrating film 46, an opening 48 is formed in each of the elements 33 on a substrate base 47 of the substrate 31. A flexible film 49 is formed all over a surface of the substrate base 47. The flexible film 49 is constructed of a silicon oxide (SiO₂) layer 51 layered on the surface of the substrate base 47, and a zirconium oxide (ZrO₂) layer 52 layered on a surface of the silicon oxide layer 51. The flexible film 49 contacts the openings 48. In this manner, a part of the flexible film 49 serves as the vibrating film 46 corresponding to the outline of the opening 48. The film thickness of the silicon oxide layer 51 can be determined based on the resonance frequency.

The lower electrode 34, the piezoelectric film 36, and the upper electrode 35 are layered on a surface of the vibrating film 46 in this order. As for the lower electrode 34, a layered film of titanium (Ti), iridium (Ir), platinum (Pt), and titanium (Ti) can be used, for example. The piezoelectric film 36 may be formed of piezoelectric zirconate titanate (PZT), for example. The upper electrode 35 may be formed of iridium (Ir), for example. Another conductive material may be used for the lower electrode 34 and the upper electrode 35. Another piezoelectric material may be used for the piezoelectric film 36.

A protective film 53 is layered on the surface of the substrate 31. The protective film 53 covers, for example, the entire surface of the substrate 31. As a result, the protective film 53 covers the element array 32, the first terminal array 39a, the second terminal array 39b, the first end 23a of the flexible printed substrate 23, and the second end 23b of the flexible printed substrate 23. For example, a silicone resin film may be used for the protective film 53. The protective film 53 protects the configuration of the element array 32, the bonding of the first terminal array 39a and the first end 23a of the flexible printed substrate 23, and the bonding of the second terminal array 39b and the second end 23b of the flexible printed substrate 23.

A partition wall 54 (one example of a partition wall section) is laid out between the adjacent openings 48. The openings 48 are partitioned by the partition wall 54. The wall thickness "t" of the partition wall 54 corresponds to the distance between the spaces of the openings 48. The partition wall 54 has two wall surfaces in planes extending in parallel to each other. The wall thickness "t" corresponds to the distance between the wall surfaces. Specifically, the wall thickness "t" can be determined by the length of a vertical line that is orthogonal to the wall surfaces and is sandwiched by the wall surfaces. The wall height "H" of the partition wall 54 corresponds to the depth of the opening 48. The depth of the opening 48 corresponds to the thickness of the substrate base 47. Therefore, the wall height "H" of the partition wall 54 can be determined by the length of the wall surface defined in the thickness direction of the substrate base 47. Since the substrate base 47 has a uniform thickness, the partition wall 54 can have a uniform wall height "H" over the entire length. When the wall thickness "t" of the partition wall 54 is decreased, the arrangement density of the vibrating film 46 can be increased. This can contribute to downsizing of the element chip 22. When the wall height "H" of the partition wall 54 is larger than the wall thickness "t" of the partition wall 54, the bending rigidity of the element chip 22 can be increased. Consequently, the distance between the openings 48 is set to be smaller than the depth of the opening 48.

A reinforcing plate (reinforcing member) 55 is fixed to a reverse surface of the substrate base 47 on the opposite side of the surface of the substrate base 47. The reverse surface of the substrate base 47 is superimposed on a surface of the reinforcing plate 55. The reinforcing plate 55 covers the openings 48 in a reverse surface of the element chip 22. The reinforcing plate 55 may have a rigid base material. For example, the reinforcing plate 55 may be formed of a silicon base plate. The plate thickness of the substrate base 47 is set to be around 100 µm. Here, the partition walls 54 are bonded to the reinforcing plate 55. The reinforcing plate 55 is bonded to each of the partition walls 54 in at least one bonding region. An adhesive can be used for bonding.

A plurality of grooves 56 are arranged on the surface of the reinforcing plate 55. The grooves 56 divide the surface of the reinforcing plate 55 into a plurality of planes 57. The plurality of planes 57 extend in a hypothetical plane HP. The reverse surface of the substrate base 47 also extends in the hypothetical plane HP. The partition walls 54 are bonded to the planes 57. The grooves 56 are recessed with respect to the hypothetical plane HP. The cross-sectional shape of the groove 56 may be a quadrangle, a triangle, a semicircle, or any other shape.

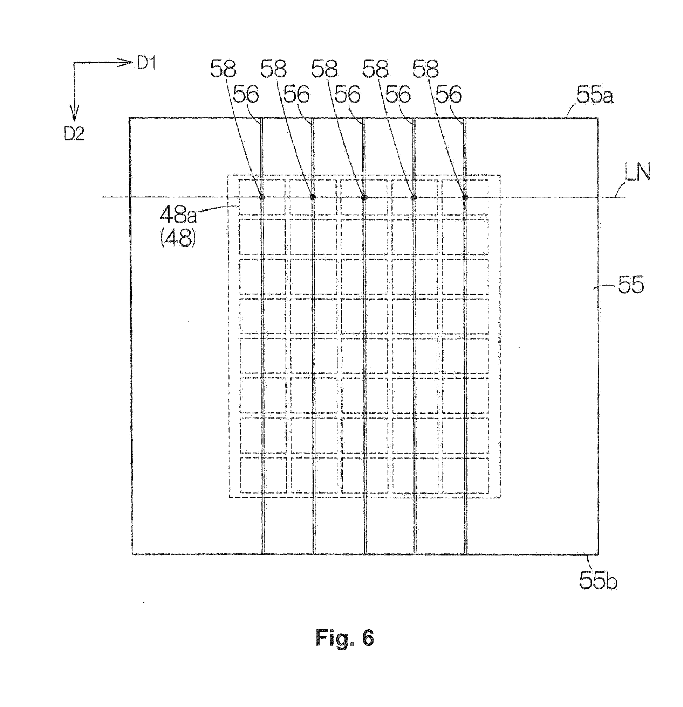

As shown in Fig. 6, the openings 48 form a line in a first direction D1. The centroids 58 of the outlines of the openings 48 are located on a straight line LN in the first direction D1 at equal pitches. Since the openings 48 are formed by copying a single outline shape, the openings 48 of the same shape are repeatedly arranged at uniform pitches. For example, an outline 48a of the opening 48 is defined as a quadrangle. More specifically, the outline 48a of the opening 48 is formed in a rectangle. The long side of the rectangle is made to coincide with the first direction D1. Since the opening 48 has the rectangular outline 48a, the partition wall 54 can have a uniform wall thickness "t" over the entire length. In such an instance, the bonding region of the partition wall 54 may be a region that includes a center position of the long side. In particular, the bonding region of the partition wall 54 may be a region that includes the entire length of the long side. The partition walls 54 may be surface-bonded to the reinforcing plate 55 with respect to the entire surface between the openings 48 over the entire length of the long side. Further, the bonding region of the partition wall 54 may be located in at least one position of each side of the quadrangle. The bonding region of the partition wall 54 may continuously surround the quadrangle. The partition walls 54 may be surface-bonded to the reinforcing plate 55 with respect to the entire surface between the openings 48 over the entire periphery of the quadrangle.

The grooves 56 are lined up in the first direction D1 in parallel to each other at equal intervals. The grooves 56 extend in a second direction D2 that intersects with the first direction D1. Both ends of the grooves 56 are open in end surfaces 55a and 55b of the reinforcing plate 55. One groove 56 crosses one column (one line) of the outlines 48a of the openings 48 in order. At least one groove 56 is connected to each opening 48. Here, the second direction D2 is orthogonal to the first direction D1. Accordingly, the groove 56 crosses the opening 48 and the partition wall 54 in the short side of the rectangle.

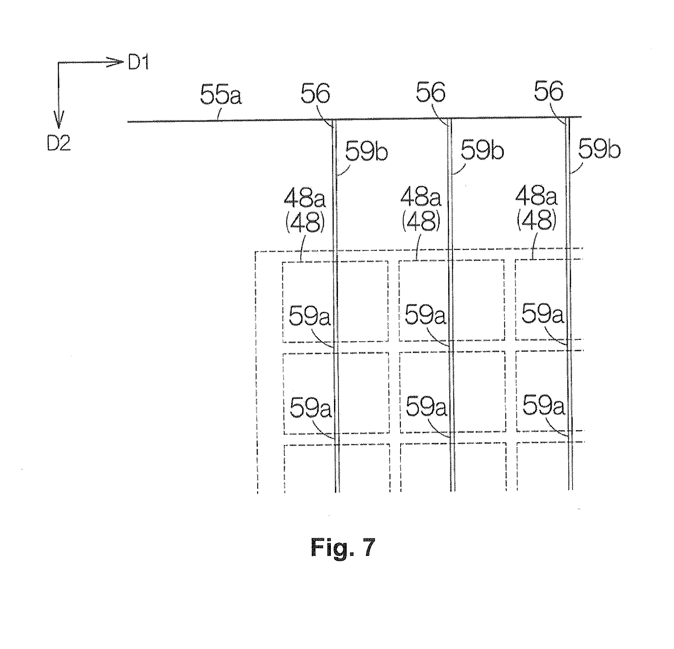

As shown in Fig. 7, the grooves 56 between the planes 57 form ventilation paths 59a and 59b between the substrate base 47 and the reinforcing plate 55. In this manner, the spaces within the grooves 56 are connected to the spaces within the openings 48. The ventilation paths 59a and 59b connect the insides and the outsides of the spaces within the openings 48. In this manner, ventilation is achieved between the spaces within the openings 48 and the outsides of the openings 48. Since one groove 56 crosses one column (one line) of the openings 48 in order, the openings 48 are connected by the ventilation paths 59a in sequence. Both ends of the grooves 56 are open in the end surfaces 55a and 55b of the reinforcing plate 55. In this manner, the ventilation paths 59b are open from the openings 48 at the column ends to the outside of the outer edge of the substrate 31.

### (2) Circuit Configuration of Ultrasonic Detection Device

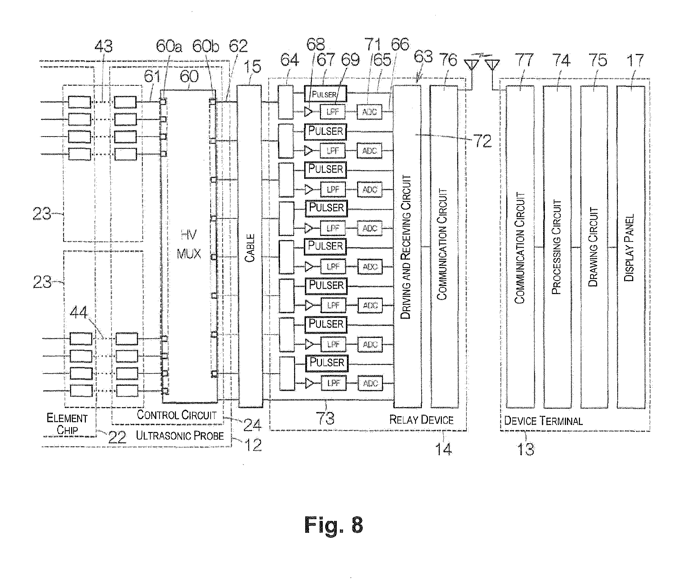

As shown in Fig. 8, a multiplexer 60 is incorporated in the control circuit 24. The multiplexer 60 has a group of ports 60a on the element chip 22 side, and a group of ports 60b on the relay device 14 side. First signal lines 61 are connected to the group of ports 60a on the element chip 22 side. The first signal lines 61 are connected to the signal lines 43 and 44 on the flexible printed substrate 23. In this manner, the first signal lines 61 are connected to the element array 32. A prescribed number of second signal lines 62 are connected to the group of ports 61b on the relay device 14 side. The prescribed number corresponds to a column number of the elements 33 in which outputting is conducted at the same time as scanning. The multiplexer 60 controls interconnection between the ports on the relay device 14 side and the ports on the element chip 22 side. The second signal lines 62 are bundled in the cable 15. The cable 15 forms a signal path with respect to each second signal line 62.

A transmitting and receiving circuit 63 is constructed in the relay device 14. The transmitting and receiving circuit 63 has a prescribed number of changing switches 64. The prescribed number corresponds to a column number of the elements 33 in which outputting is conducted at the same time as scanning. Each of the changing switches 64 is connected to the cable 15. Each of the changing switches 64 is connected to each of the second signal lines 62.

The transmitting and receiving circuit 63 has a transmission path 65 and a reception path 66 for each of the changing switches 64. The transmission path 65 and the reception path 66 are connected to the changing switch 64 in parallel. The changing switch 64 selectively connects the transmission path 65 and the reception path 66 to the multiplexer 60. A pulser 67 is incorporated in the transmission path 65. The pulser 67 outputs a pulse signal at a frequency corresponding to the resonance frequency of the vibrating film 46. An amplifier 68, a low-pass filter (LPF) 69, and an analog-digital converter (ADC) 71 are incorporated in the reception path 66. A detection signal of each of the elements 33 is amplified, and converted into a digital signal.

The transmitting and receiving circuit 63 has a driving/receiving circuit 72. The transmission path 65 and the reception path 66 are connected to the driving/receiving circuit 72. The driving/receiving circuit 72 controls the pulser 67 simultaneously depending on the state of scanning. The driving/receiving circuit 72 receives a digital signal of a detection signal depending on the state of scanning. The driving/receiving circuit 72 is connected to the multiplexer 60 through a control line 73. The multiplexer 60 conducts control of interconnection based on a control signal supplied from the driving/receiving circuit 72.

A processing circuit 74 is incorporated in the device terminal 13. The processing circuit 74 can be provided with a central processing unit (CPU) 74 and a memory, for example. The entire operation of the ultrasonic diagnostic device 11 is controlled in accordance with processing of the processing circuit 74. The processing circuit 74 controls the driving/receiving circuit 72 in accordance with instructions input by a user. The processing circuit 74 generates an image in accordance with a detection signal of the element 33. The image is specified by drawing data.

A drawing circuit 75 is incorporated in the device terminal 13. The drawing circuit 75 is connected to the processing circuit 74. The display panel 17 is connected to the drawing circuit 75. The drawing circuit 75 generates a driving signal in accordance with drawing data generated in the processing circuit 74. The driving signal is sent to the display panel 17. As a result, an image is displayed on the display panel 17.

Communication circuits 76 and 77 are incorporated in the relay device 14 and the device terminal 13, respectively. In the relay device 14, the communication circuit 76 is connected to the driving/receiving circuit 72. In the device terminal 13, the communication circuit 77 is connected to the processing circuit 74. The communication circuits 76 and 77 can communicate with each other wirelessly. The driving/receiving circuit 72 is connected to the processing circuit 74 through wireless communication.

### (3) Operation of Ultrasonic Detection Device

Next, the operation of the ultrasonic diagnostic device 11 will be explained briefly. The processing circuit 74 gives the driving/receiving circuit 72 instructions to transmit and receive ultrasonic waves. The driving/receiving circuit 72 supplies a control signal to the multiplexer 60, and supplies a driving signal to each of the pulsers 67. The pulser 67 outputs a pulse signal in response to the supply of the driving signal. The multiplexer 60 connects the port of the group of ports 60a to the port of the group of ports 60b in response to the instructions of the control signal. The pulse signal is supplied to the elements 33 per column through the lower electrode terminals 41 and the upper electrode terminals 42 in response to the selection of the port. The vibrating film 46 vibrates in response to the supply of the pulse signal. As a result, desired ultrasonic waves are emitted toward a target (for example, the inside of a human body).

After ultrasonic waves are transmitted, the changing switch 64 is switched. The multiplexer 60 maintains the connection relation of the ports. The changing switch 64 establishes a connection between the reception path 66 and the second signal line 62 instead of a connection between the transmission path 65 and the second signal line 62. Reflected waves of ultrasonic waves vibrate the vibrating film 46. As a result, a detection signal is output from the element 33. The detection signal is converted into a digital signal, and sent into the driving/receiving circuit 72.

Transmission and reception of ultrasonic waves are repeated. For repeating transmission and reception of ultrasonic waves, the multiplexer 60 changes the connection relation of the ports. As a result, line scanning or sector scanning is achieved. When scanning is finished, the processing circuit 74 generates an image based on the digital signal of the detection signal. The generated image is displayed on the screen of the display panel 17.

In the ultrasonic probe 12, the element chip 22 and the flexible printed substrate 23 form an annular body in cooperation. The flexible printed substrate 23 is not extracted along a two-dimensional plane that is continuous with the element chip 22, but is extracted three-dimensionally along the space 29 so as to surround the space 29. As a result, it is possible to reduce the two-dimensional size. The element chip 22 can contact an object without being hindered by the flexible printed substrate 23. In particular, the annular body constructs a finger band. A finger Fi can enter the internal space 28 of the tubular body 25. As a result, the annular body can be mounted on a fingertip Fi (for example, a distal joint, or a distal joint and a proximal joint). The tubular body 25 can be closely attached to a finger Fi due to the action of an elastic force. Consequently, the element chip 22 can be fixed to a fingertip. The bend of a finger Fi can be allowed because there is no bundle of signal lines along the two-dimensional plane that is continuous with the element chip 22. At least a part of the annular body unit 21 (here, the element chip 22) can transmit a contact state to a fingertip Fi. A feel of an object can be transmitted to a fingertip Fi through the element chip 22. A user can sense a feel of a diseased part while mounting the ultrasonic probe 12.

In the element chip 22, the element 33 can be formed to be thin. The element 33 can be formed on the thin substrate 31. Even in a case where the reinforcing plate 55 is fixed to the substrate 31, the element chip 22 can be formed to be thin. At the same time, the reinforcing plate 55 reinforces the strength of the substrate 31. In particular, since the wall thickness "t" is smaller than the wall height "H" in the partition wall 54, the sufficient rigidity of the partition wall 54 can be obtained in the thickness direction of the substrate 31 due to the section modulus. Force in the thickness direction of the substrate 31 is transmitted through the partition wall 54 and supported by the reinforcing plate 55. In this manner, the element chip 22 has sufficient strength in the thickness direction of the substrate 31. Accordingly, even when the plate thickness of the substrate base 47 is set to be around 100 µm, for example, the reinforcing plate 55 can prevent the substrate base 47 from being damaged. On the other hand, in a case where the element array is constructed of a bulk-type ultrasonic transducer element, the plate thickness of the substrate is set to be around several millimeters. Even when the reinforcing plate 55 is bonded, the thickness of the element chip 22 can be reduced securely compared to the case where the element array is constructed of a bulk-type ultrasonic transducer element. In addition, since the acoustic impedance of the vibrating film 46 is close to that of a human body compared to a bulk-type ultrasonic transducer element, an acoustic impedance matching layer can be omitted in the element chip 22 unlike in the case of a bulk-type ultrasonic transducer element. Omission of the matching layer can further contribute to making the element chip 22 thinner. As a result, the element chip 22 perfect for a finger band can be achieved.

The reinforcing plate 55 is bonded to each of the partition walls 54 in at least one bonding region. When the partition walls 54 are bonded to the reinforcing plate 55, the movement of the partition walls 54 is restricted by the reinforcing plate 55. Thus, vibration of the partition walls 54 can be prevented. As a result, crosstalk between the elements 33 can be prevented. Further, when the movement of the partition walls 54 is restricted, vibration of the partition walls 54 can be prevented from acting on ultrasonic vibration of the elements 33. Then, ultrasonic vibration in a clear vibration mode can be obtained in the elements 33. When vibration of the partition walls 54 is avoided, the amplitude of ultrasonic vibration can be prevented from being decreased. On the other hand, when the partition wall 54 moves, a distorted vibration mode having a lower frequency than the vertical vibration mode of the vibrating film 46 occurs. Furthermore, the kinetic energy of the vibrating film 46 decreases by the movement of the partition wall 54, and the amplitude of the vibration decreases.

Although the internal space of the opening 48 is surrounded by the substrate 31, the flexible film 49 (the vibrating film 46) and the reinforcing plate 55, the groove 56 secures ventilation between the internal space of each opening 48 and the outside of the internal space. Consequently, the internal space of the opening 48 is not sealed. The internal space of the opening 48 can easily follow change in the ambient pressure. It is thus possible to securely prevent the element 33 from being damaged. If the internal space of the opening 48 is hermetically sealed, there is a concern that the ultrasonic transducer element will be damaged due to change in the pressure.

The bonding region of the partition walls 54 can be a region that includes a center position of the long side. Therefore, a part of the partition walls 54 in which the amplitude of vibration is large is bonded to the reinforcing plate 55. As a result, vibration of the partition walls 54 can be effectively prevented. Also, the bonding region of the partition walls 54 can be a region that includes the entire length of the long side. When the partition walls 54 are bonded to the reinforcing plate 55 over the entire length of the long side, vibration of the partition walls 54 can be securely prevented. Further, the partition walls 54 can be surface-bonded with respect to the entire surface between the openings 48 over the entire length of the long side. When the partition walls 54 are surface-bonded to the reinforcing plate 55 with respect to the entire surface between the openings 48 over the entire length of the long side, vibration of the partition walls 54 can be securely prevented.

It is sufficient that the bonding region of the partition walls 54 is located in at least one position of each side of the quadrangle. When the partition walls 54 are bonded to the reinforcing plate 55 in each side of the quadrangle, vibration of the partition walls 54 can be securely prevented. Also, the bonding region of the partition walls 54 can continuously surround the quadrangle. When the partition walls 54 are bonded to the reinforcing plate 55 with respect to the entire region of the quadrangle, vibration of the partition walls 54 can be securely prevented. Further, the partition walls 54 can be surface-bonded with respect to the entire surface between the openings 48 over the entire periphery of the quadrangle. When the partition walls 54 are surface-bonded to the reinforcing plate 55 with respect to the entire surface between the openings 48 over the entire periphery of the quadrangle, vibration of the partition walls 54 can be securely prevented.

In the ultrasonic probe 12, the multiplexer 60 is incorporated in the control circuit 24. The second signal lines 62 are extracted from the multiplexer 60. Compared to the case where the first signal lines 61 are extracted directly from the ultrasonic probe 12, the number of the extracted signal lines is decreased. As a result, the size of the cable 15 connected to the ultrasonic probe 12 can be reduced. Consequently, size reduction of the ultrasonic probe 12 can be promoted, and the bend of a finger Fi is not hindered irrespective of wiring of the cable 15.

### (4) Annular Body Unit according to Second Embodiment

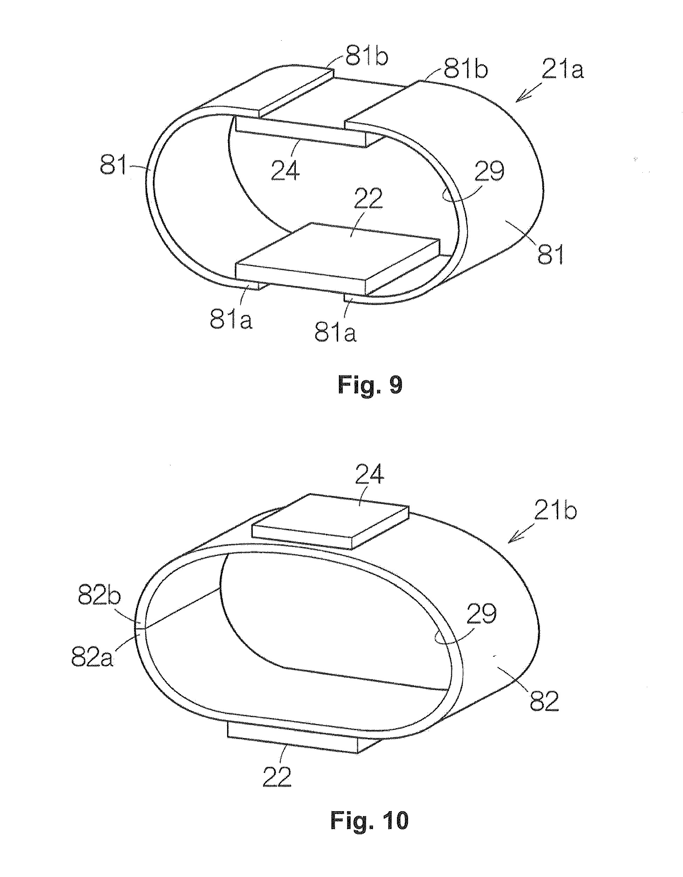

Fig. 9 schematically shows an annular body unit 21a according to a second embodiment of the present invention. The ultrasonic diagnostic device 11 can use the annular body unit 21a instead of the above-described annular body unit 21. In this second embodiment, the annular body is constructed of the element chip 22 and a pair of flexible printed substrates 81 (examples of a first flexible wiring section and a second flexible wiring section). Each flexible printed substrate 81 is connected to the element chip 22 at a first end 81a, and connected to the control circuit 24 at a second end 81b. The element chip 22, the flexible printed substrates 81, and the control circuit 24 collectively surround the space 29. The other configurations are similar to those of the first embodiment. The configurations or structures that are equivalent to those of the first embodiment are given the same reference numerals and the overlapping explanations are omitted. In the same manner as described above, a flexible wiring member that forms an annular body with a supporting member for supporting a conductive line, or a flexible wiring member that forms an annular body with an electric wire in which a conductive line is covered with an insulating body can be used instead of the flexible printed substrates 81.

### (5) Annular Body Unit according to Third Embodiment

Fig. 10 schematically shows an annular body unit 21b according to a third embodiment of the present invention. The ultrasonic diagnostic device 11 can use the annular body unit 21b instead of the above-described annular body unit 21. In this third embodiment, the annular body is constructed of a single flexible printed substrate 82 alone. A first end 82a of the flexible printed substrate 82 is connected to a second end 82b of the flexible printed substrate 82. The flexible printed substrate 82 surrounds the space 29 alone. The element chip 22 and the control circuit 24 are mounted on a surface of the flexible printed substrate 82. In order to connect the lower electrode terminals 41 and the upper electrode terminals 42 to the signal lines 43 and 44 on the flexible printed substrate 82 in the element chip 22, conductive via holes may be formed in the flexible film 49, the substrate base 47 and the reinforcing plate 55 such that the conductive via holes penetrate through the flexible film 49, the substrate base 47 and the reinforcing plate 55. The other configurations are similar to those of the first embodiment and the second embodiment. The configurations or structures that are equivalent to those of the first embodiment are given the same reference numerals and the overlapping explanations are omitted. In the same manner as described above, a flexible wiring member that forms an annular body with a supporting member for supporting a conductive line, or a flexible wiring member that forms an annular body with an electric wire in which a conductive line is covered with an insulating body can be used instead of the flexible printed substrate 82.

### (6) Annular Body Unit according to Fourth Embodiment

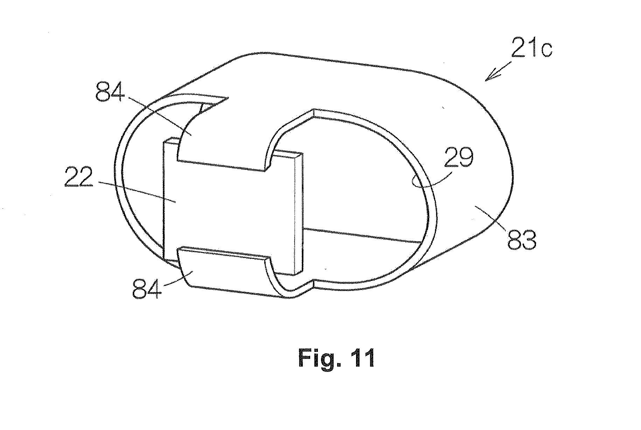

Fig. 11 schematically shows an annular body unit 21c according to a fourth embodiment of the present invention. The ultrasonic diagnostic device 11 can use the annular body unit 21c instead of the above-described annular body unit 21. In this fourth embodiment, the annular body is constructed of a single flexible printed substrate 83 alone. The flexible printed substrate 83 has a pair of connecting pieces 84 that extend from the annular body in a direction orthogonal to a circumferential direction of the annular body and located in opposed positions of the annular body. The element chip 22 is connected between tip ends of the connecting pieces 84. When a finger Fi of an operator enters the space 29 of the annular body, the element chip 22 can be positioned at the tip end of the finger Fi. Although not shown in Figure 11, the control circuit 24 is preferably connected to the flexible printed substrate 83 in the same manner as described in the first embodiment with reference to Figure 3. The other configurations are similar to those of the first embodiment. The configurations or structures that are equivalent to those of the first embodiment are given the same reference numerals and the overlapping explanations are omitted. In the same manner as described above, a flexible wiring member that forms an annular body with a supporting member for supporting a conductive line, or a flexible wiring member that forms an annular body with an electric wire in which a conductive line is covered with an insulating body can be used instead of the flexible printed substrate 83.

### (7) Circuit Configuration according to Another Embodiment

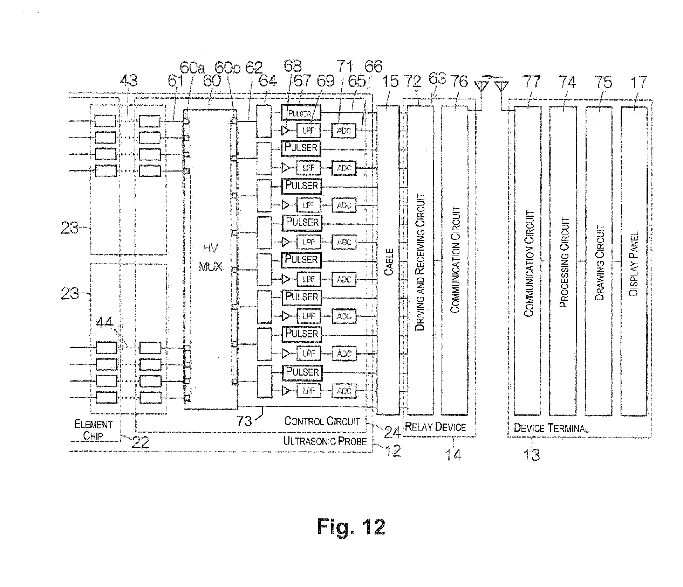

Fig. 12 schematically shows a circuit configuration according to another embodiment. In this embodiment, the changing switch 64, the transmission path 65 and a reception path 66 are incorporated in the control circuit 24 in addition to the multiplexer 60. With this circuit configuration, a digital signal can be communicated in the cable 15. Therefore, a noise resistance to exogenous noise can be increased compared to communication of an analog signal.

### (8) Ultrasonic Probe according to Another Embodiment

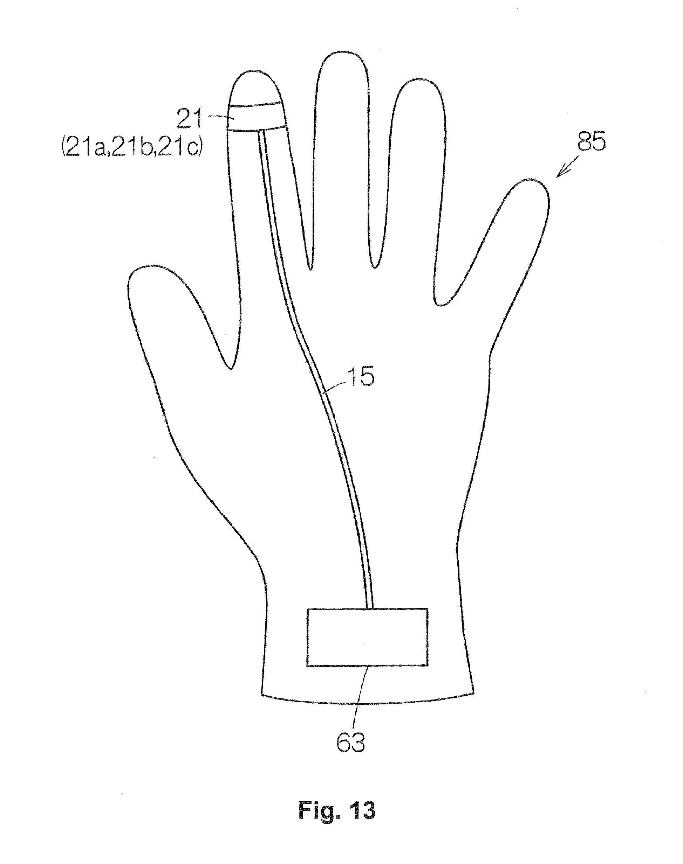

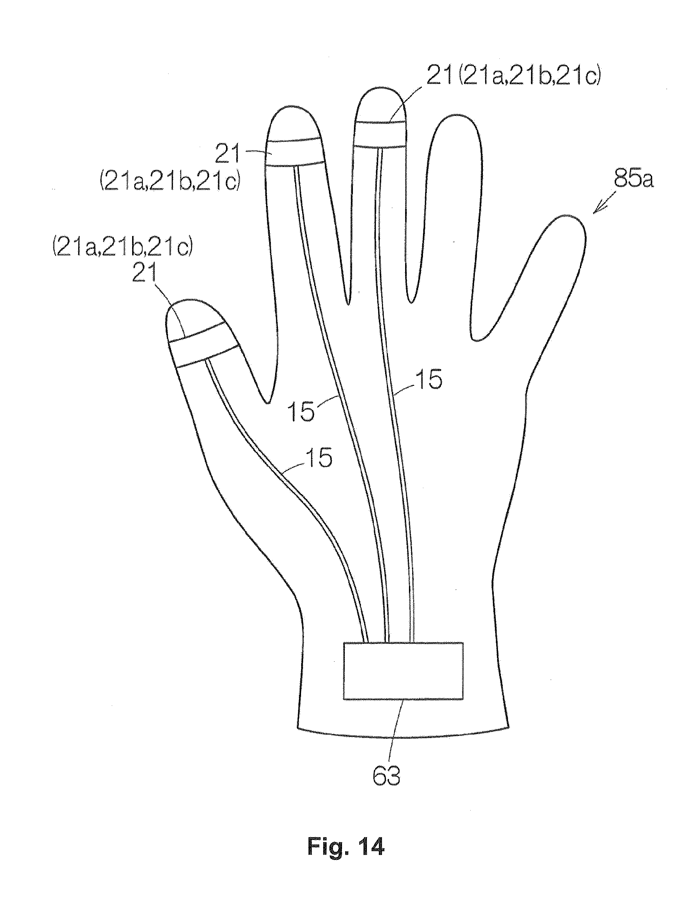

Fig. 13 schematically shows an ultrasonic probe 85 according to another embodiment. The ultrasonic diagnostic device 11 can use the ultrasonic probe 85 instead of the above-described ultrasonic probe 12. The ultrasonic probe 85 forms a glove. The glove is made of an elastic film body. As the material for the film body, for example, a resin material having flexibility can be used. For example, the index finger of the glove serves as the tubular body 25. The annular body unit 21 is embedded into the film of the tubular body 25. The annular body unit 21 is constructed as a finger band. The annular body unit 21 may be replaced with the annular body unit 21a, 21b or 21c. The transmitting and receiving circuit 63 is embedded into the film of the wrist of the glove. The transmitting and receiving circuit 63 is connected to the control circuit 24 of the annular body unit 21 by the cable 15. For example, the cable 15 can be embedded into the film of the glove. The other configurations can be made similar to those of the above-described embodiments. The configurations or structures that are equivalent to those of the above-described embodiments are given the same reference numerals and the overlapping explanations are omitted. Alternatively, in an ultrasonic probe 85a shown in Fig. 14, a plurality of annular body units 21 (21a, 21b or 21c) may be embedded into fingers of a glove.

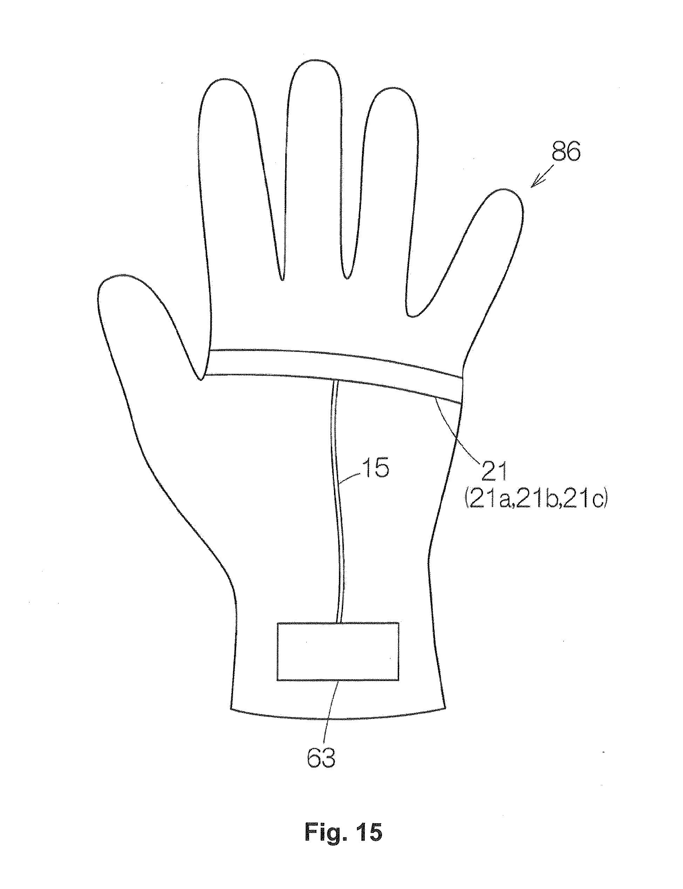

Fig. 15 schematically shows an ultrasonic probe 86 according to yet another embodiment. The ultrasonic probe 86 forms a glove similarly to the ultrasonic probe 85. The body of the glove serves as the tubular body 25. The annular body unit 21 is embedded into the film of the tubular body 25. The element chip 22 is disposed in the palm or the back of the hand. The annular body unit 21 may be replaced with the annular body unit 21a, 21b or 21c. The transmitting and receiving circuit 63 is embedded into the film of the wrist of the glove. The transmitting and receiving circuit 63 is connected to the control circuit 24 of the annular body unit 21 by the cable 15. For example, the cable 15 can be embedded into the film of the glove. The other configurations can be made similar to those of the above-described embodiments. The configurations or structures that are equivalent to those of the above-described embodiments are given the same reference numerals and the overlapping explanations are omitted.

In the ultrasonic probe 86, the second to the fifth metacarpal bones can enter the internal space 28 of the tubular body 25. As a result, the annular body can be mounted on the second to the fifth metacarpal bones. The tubular body 25 can be closely attached to the hand due to the action of an elastic force. Consequently, the element chip 22 can be fixed to the palm or the back of the hand. The bend of the hand can be allowed because there is no bundle of signal lines along the two-dimensional plane that is continuous with the element chip 22.

While the present embodiment has been explained in detail as above, it will be apparent to those skilled in the art that various changes and modifications can be made herein without substantially departing from the subject matter and the effect of the present invention. Therefore, such changes and modifications are included in the scope of the invention. For example, the terms used in the specification or the drawings at least once together with a different term having a broader or similar meaning can be replaced with the different term in any portion of the specification or the drawings. Also, the configurations and the operations of the ultrasonic diagnostic device 11, the ultrasonic probe 12, the element chip 22, the ultrasonic transducer element 33 and the like are not limited to the present embodiment, and various changes and modifications are possible.

### GENERAL INTERPRETATION OF TERMS

In understanding the scope of the present invention, the term "comprising" and its derivatives, as used herein, are intended to be open ended terms that specify the presence of the stated features, elements, components, groups, integers, and/or steps, but do not exclude the presence of other unstated features, elements, components, groups, integers and/or steps. The foregoing also applies to words having similar meanings such as the terms, "including", "having" and their derivatives. Also, the terms "part," "section," "portion," "member" or "element" when used in the singular can have the dual meaning of a single part or a plurality of parts. Finally, terms of degree such as "substantially", "about" and "approximately" as used herein mean a reasonable amount of deviation of the modified term such that the end result is not significantly changed. For example, these terms can be construed as including a deviation of at least ± 5% of the modified term if this deviation would not negate the meaning of the word it modifies.

While only selected embodiments have been chosen to illustrate the present invention, it will be apparent to those skilled in the art from this disclosure that various changes and modifications can be made herein without departing from the scope of the invention as defined in the appended claims. Furthermore, the foregoing descriptions of the embodiments according to the present invention are provided for illustration only, and not for the purpose of limiting the invention as defined by the appended claims and their equivalents.

## Claims

1. An ultrasonic probe comprising:
an element chip including a substrate forming a plurality of openings arranged in an array pattern and a plurality of ultrasonic transducer elements disposed at the openings;
a flexible wiring member connected to the element chip, and forming at least a part of an annular body surrounding a space; and
a control circuit connected to the flexible wiring member and electrically connected to the ultrasonic transducer elements via the flexible wiring member.

2. The ultrasonic probe according to claim 1, wherein
the flexible wiring member and the element chip collectively form the annular body.

3. The ultrasonic probe according to claim 1, wherein
the flexible wiring member forms the annular body.

4. The ultrasonic probe according to any of claims 1-3, further comprising a control circuit chip including the control circuit, and
the flexible wiring member including
a first flexible wiring section connected to the element chip and extending from the element chip in a first direction, and
a second flexible wiring section connected to the element chip and extending from the element chip in a second direction that is opposite to the first direction,
the element chip, the first flexible wiring section, the second flexible wiring section, and the control circuit chip collectively forming the annular body.

5. The ultrasonic probe according to any of claims 1-4, further comprising
a tubular body member made of an elastic film body and defining a tubular internal space passing through the space surrounded by the annular body to form an entrance space for a finger, with the annular body constituting a finger band.

6. The ultrasonic probe according to any of claims 1-5, wherein
the annular body constitutes a finger band, and at least a part of the annular body conveys a contact state to a finger.

7. The ultrasonic probe according to any of claims 1-6, further comprising
a reinforcing member fixed to a second surface of the substrate, which is an opposite side of a first surface where the ultrasonic transducer elements are disposed, for reinforcing the substrate.

8. The ultrasonic probe according to any of claims 1-7, wherein
internal spaces of the openings are continuous with an external space of the substrate.

9. The ultrasonic probe according to claim 7 or claim 8, wherein
the reinforcing member is bonded to a partition wall section of the substrate between the openings in at least one bonding region.

10. The ultrasonic probe according to any of claims 1-9, wherein
the control circuit includes a multiplexer configured to control traffic of signals between a first prescribed number of first signal lines extracted from the ultrasonic transducer elements and a second prescribed number of second signal lines, the second prescribed number being smaller than the first prescribed number.

11. An electronic instrument comprising:
the ultrasonic probe according to any of claims 1-10; and
a processing circuit connected to the ultrasonic probe, and configured to process output signals of the ultrasonic transducer elements.

12. An ultrasonic diagnostic device comprising:
the ultrasonic probe according to any of claims 1-10;
a processing circuit connected to the ultrasonic probe, and configured to process output signals of the ultrasonic transducer elements to generate an image; and
a display device configured to display the image.

13. An ultrasonic probe set comprising:
the ultrasonic probe according to any of claims 1-10; and
a relay device connected to the ultrasonic probe, and including a communication circuit connected to the control circuit.

14. The ultrasonic probe set according to claim 13, wherein
the relay device includes at least a part of the control circuit.

15. An electronic instrument comprising:
the ultrasonic probe set according to claim 13 or claim 14; and
a processing circuit wirelessly connected to the communication circuit, and configured to process output signals of the ultrasonic transducer elements.

16. An ultrasonic diagnostic device comprising:
the ultrasonic probe set according to any of claims 13-15;
a processing circuit wirelessly connected to the communication circuit, and configured to process output signals of the ultrasonic transducer elements to generate an image; and
a display device configured to display the image.
